(19) Europäisches Patentamt
European Patent Office
Office européen des brevets

(11) Veröffentlichungsnummer: **0 176 924**
**B1**

(12) **EUROPÄISCHE PATENTSCHRIFT**

(45) Veröffentlichungstag der Patentschrift: **31.05.89**

(51) Int. Cl.⁴: **C 07 C 97/10**, C 07 D 295/10

(21) Anmeldenummer: **85112068.3**

(22) Anmeldetag: **24.09.85**

# (54) Verfahren zur Herstellung von Beta-Aminoethylketonen.

(30) Priorität: **05.10.84 DE 3436450**

(43) Veröffentlichungstag der Anmeldung: **09.04.86 Patentblatt 86/15**

(45) Bekanntmachung des Hinweises auf die Patenterteilung: **31.05.89 Patentblatt 89/22**

(84) Benannte Vertragsstaaten: **CH DE FR GB IT LI**

(56) Entgegenhaltungen:
**EP-A-0 004 000**
**DE-C-379 950**

**PATENT ABSTRACTS OF JAPAN, Band 8, Nr. 122 (C227) 1559, 8. Juni 1984; & JP - A - 59 33276 (HOKURIKU SEIYAKU) 23.02.1984**

(73) Patentinhaber: **BAYER AG, D-5090 Leverkusen 1 Bayerwerk (DE)**

(72) Erfinder: **Seng, Florin, Dr., Am Katterbach 46, D-5060 Bergisch- Gladbach 2 (DE)**
Erfinder: **Bremen, Josef, Dr., Am Kettnersbusch 1e, D-5090 Leverkusen 3 (DE)**

**EP 0 176 924 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der Erfindung ist ein Verfahren zur Herstellung von α-Aminoethylketonen durch Kondensation von H-aciden Arylketonen mit Aldehyden und Ammoniak bzw. Aminen im Sinne einer "Mannich-Reaktion".

Es ist allgemein bekannt (vgl. z. B. "Synthesis" 1973, 703 ff und "Houben-Weyl" 11/I (1957), 731 ff), daß diese Kondensation in Gegenwart von Salzsäure in wäßrigem oder (wäßrig)-alkoholischem Medium durchgeführt wird, wobei die Säure zweckmäßigerweise in Form der Hydrochloride der als Reaktionspartner verwendeten Amine eingesetzt wird.

Auch bei dem in EP-A-0 004 000 beschriebenen Verfahren zur Herstellung neuer Propanon-Derivate wird in einem azeotropen Reaktionsgemisch ausschließlich mit Salzsäure als Katalysator gearbeitet.

Diese an sich bewährten Methoden weisen indessen den Nachteil auf, daß als Nebenprodukt der HCl-katalysierten Mannich-Kondensation die sehr toxischen Chlormethylether entstehen können.

Weiterhin ist bekannt, daß sich die aromatischen β-Aminoketone auch durch Umsetzung der entsprechenden β-Halogenketone mit Aminen herstellen lassen.

Auch dieses Verfahren ist nicht optimal, da die als Ausgangsmaterial eingesetzten β-Halogenketone zu Hautreizungen und Allergien führen können.

Aufgabe der vorliegenden Erfindung war es daher, ein Verfahren zur Verfügung zu stellen, daß die vorstehend genannten Nachteile nicht aufweist.

Erfindungsgemäß wird diese Aufgabe dadurch gelöst, daß man die Mannich-Kondensation in organischen Nitrilen oder Glykolethern in Gegenwart von Sulfonsäuren bzw. im Falle der Glykolether auch in Gegenwart von Schwefelsäure durchführt.

Es muß als ausgesprochen überraschend angesehen werden, daß die Reaktion auch unter diesen Bedingungen sehr glatt abläuft und gute Ausbeuten liefert, da in den üblichen wäßrig alkoholischen Lösungsmitteln die erfindungsgemäß einzusetzenden Säurekatalysatoren versagen.

Das neue Verfahren eignet sich besonders zur Herstellung von Verbindungen der Formel

$[Ar\text{-}CO\text{-}CH_2\text{-}CH_2\text{-}]_n\ NR_{3\text{-}n} \cdot R'SO_3H$

worin

- Ar einen Arylrest,
- R Wasserstoff, Alkyl, Aralkyl oder Cycloalkyl,
- R' OH, Alkyl oder Aryl und
- n die Zahlen 1 oder 2 bedeuten, mit der Maßgabe, daß R für Alkyl steht oder 2 Reste R gemeinsam mit dem N-Atom die restlichen Glieder eines gesättigten Heterocyclus bilden, wenn n die Zahl 1 bedeutet.

Geeignete Reste Ar sind Carbocyclische aromatische Reste, wie z. B. solche der Naphthalin- und Benzolreihe. Bevorzugt sind Phenylreste, die gegebenenfalls substituiert sind, beispielsweise durch Cl, $-CH_3$ und -Phenyl.

Geeignete Alkylreste R/R' sind solche mit 1 - 6 C-Atomen, die ebenfalls substituiert sein können, z. B. durch -OH oder $-OC_1$ - $C_4$-Alkyl.

Geeignete Aralkylreste sind Benzyl und Phenylethyl.

Geeignete Cycloalkylreste sind Cyclohexylreste.

Geeignete Arylreste R' sind gegebenenfalls durch $CH_3$ oder Cl substituierte Phenylreste.

Geeignete Heterocyclen, die zwei Reste R bilden können

sind: 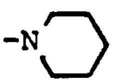 und 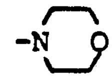 .

Das erfindungsgemäße Verfahren wird im übrigen unter den üblichen Bedingungen einer Mannich-Reaktion durchgeführt.

Bevorzugt einzusetzender Aldehyd ist Formaldehyd. Geeignete Amine sind z. B.: Methyl-, Ethyl-, n-Propyl-, Dimethyl-, Diethyl- und Cyclohexylamin sowie Piperidin und Morpholin.

Als Lösungsmittel seien beispielhaft genannt: Acetonitril, Propionitril, Glykolmonomethylether und Propylenglykolmonomethylether.

Die Reaktionstemperaturen liegen im allgemeinen zwischen 80 und 130°C.

Bevorzugte Sulfonsäuren sind Methan-Benzol- und p-Toluolsulfonsäure.

Von den Säurekatalysatoren (d. h. entweder Schwefelsäure oder die Sulfonsäuren) werden mindestens 1 Mol pro Mol Amin eingesetzt, wobei darauf zu achten ist, daß zuerst Katalysator und Amin in Kontakt gebracht werden, bevor die übrigen Reaktionspartner hinzugefügt werden. Zweckmäßigerweise verwendet man daher wie bei der klassichen Mannich-Reaktion die entsprechenden Aminsalze.

Die den neuen Verfahrensprodukten der oben angegebenen Formel zugrundeliegenden Basen sind zum großen Teil bekannt und stellen wertvolle Zwischenprodukte zur Herstellung von optischen Aufhellern vom Pyrazolintyp dar (vgl. z. B. US-A-3 133 080).

**Beispiel 1**

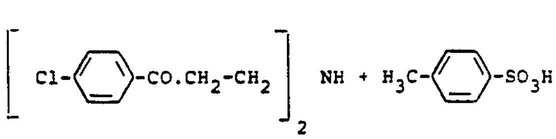

30,9 g (0,2 Mol) 4-Chloracetophenon werden in 50 ml Acetonitril vorgelegt. Man versetzt mit 13,6 g

(0,2 Mol) 25 %-igen wäßrigem Ammoniak und 57 g (0,3 Mol) p-Toluolsulfonsäure und rührt 15 min. Danach gibt man 6 g (0,2 Mol) Paraformaldehyd zu und rührt 10 Std. bei 80° C. Beim anschließenden Abkühlen auf 0° C fallen 51 g Rohprodukt aus. Durch Ausrühren in 200 ml Aceton erhält man 42 g (80 % d. Th.) Bis-[β-4-chlorbenzoyl-ethyl]ammoniumtoluolsulfonat vom Schmp.: 190 - 191° C.

In analoger Weise erhält man:

2) 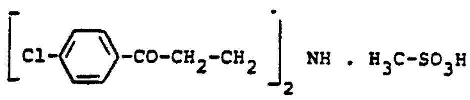

3) 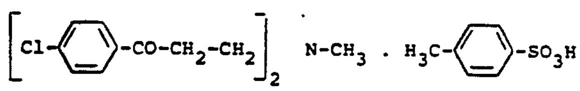

4) 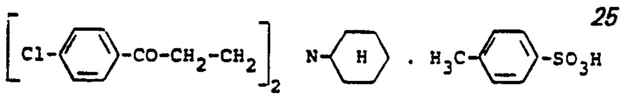

5) 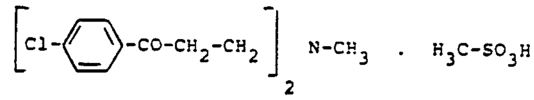

6) 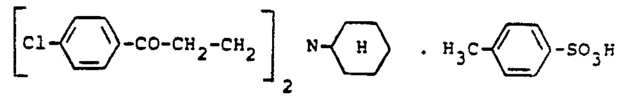

7) 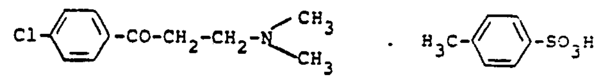

8) 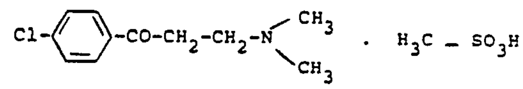

9) 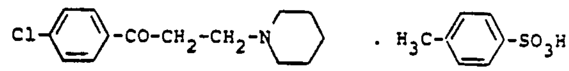

10) 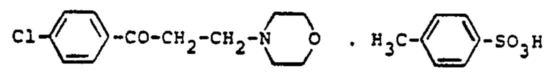

11) 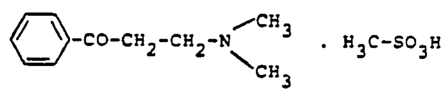

12) 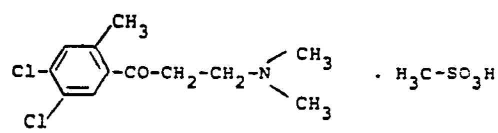

13) 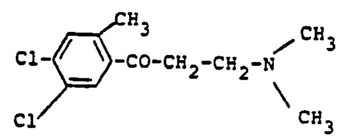 · 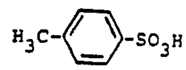

14) 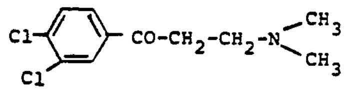 · $H_3C-SO_3H$

**Beispiel 15**

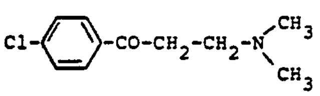 · $H_2SO_4$

309 g (2 Mol) 4-Chloracetophenon werden in 300 ml Propylenglykolmonomethylether vorgelegt. Man versetzt mit 520 g (5,2 Mol) konz. Schwefelsäure und 468 g (5,2 Mol) 50 %-iger wäßriger Dimethylaminlösung und rührt 15 min.

Danach gibt man 78 g (2,6 Mol) Paraformaldehyd zu und rührt 3 Std. bei 110 - 120° C.

Anschließend wird das Lösungsmittel im Wasserstrahlvakuum abdestilliert und der hinterbliebende feste Rückstand in 1 l Aceton angerührt. Man erhält 468 g β-4-Chlorbenzoyl-ethyl-dimethylammoniumsulfat vom Schmp. 120 - 122° C. Aus dem Filtrat scheiden beim Stehen über Nacht weitere 100 g Produkt vom Schmp. 118 - 120° C ab.

**Patentansprüche**

1. Verfahren zur Herstellung von β-Aminoethylketonen durch Kondensation von H-aciden Arylketonen mit Aldehyden und Ammoniak bzw. Aminen, dadurch gekennzeichnet, daß man die Reaktion in organischen Nitrilen oder Glykolethern in Gegenwart von Sulfonsäuren bzw. - im Falle der Glykolether - auch in Gegenwart von Schwefelsäure durchführt.

2. Verfahren nach Anspruch 1, dadurch gekennzeichnet, daß man das Ammoniak bzw. die Amine in Form der schwefel- oder sulfonsäuren Salze einsetzt.

3. Verfahren nach Anspruch 1, dadurch gekennzeichnet, daß man die Reaktion in Acetonitril bzw. Propylenglykolmonomethylether durchführt.

4. Verfahren nach Anspruch 1, dadurch gekennzeichnet, daß man als Aldehyd Formaldehyd einsetzt.

5. Verfahren nach Anspruch 1, dadurch gekennzeichnet, daß man als Sulfonsäuren Benzol-, p-Toluol- oder Methansulfonsäure einsetzt.

**Claims**

1. Process for the preparation of β-aminoethyl ketones by condensation of H-acid aryl ketones with aldehydes and ammonia or amines, characterised in that the reaction is carried out in organic nitriles or glycol ethers in the presence of sulphonic acids or - in the case of the glycol ethers - also in the presence of sulphuric acid.

2. Process according to Claim 1, characterised in that the ammonia and the amines are employed in the form of sulphuric acid or sulphonic acid salts.

3. Process according to Claim 1, characterised in that the reaction is carried out in acetonitrile or propylene glycol monomethyl ether.

4. Process according to Claim 1, characterised in that formaldehyde is employed as the aldehyde.

5. Process according to Claim 1, characterised in that benzene-, p-toluene- or methanesulphonic acid is employed as the sulphonic acid.

**Revendications**

1. Procédé de production de β-amino-éthylcétones par condensation d'arylcétones à hydrogène acide avec des aldéhydes et l'ammoniac ou des amines, caractérisé en ce qu'on conduit la réaction dans des nitriles organiques ou dans des éthers de glycols en présence d'acides sulfoniques ou aussi - dans le cas des éthers de glycols - en présence d'acide sulfurique.

2. Procédé suivant la revendication 1, caractérisé en ce qu'on utilise l'ammoniac ou les amines sous la forme des sels d'acide sulfurique ou d'acide sulfonique.

3. Procédé suivant la revendication 1, caractérisé en ce qu'on conduit la réaction dans l'acétonitrile ou dans l'éther monométhylique du propylène-glycol.

4. Procédé suivant la revendication 1, caractérisé en ce qu'on utilise comme aldéhyde le formaldéhyde.

5. Procédé suivant la revendication 1, caractérisé en ce qu'on utilise comme acides sulfoniques l'acide benzènesulfonique, p-toluènesulfonique ou méthanesulfonique.